(19) Europäisches Patentamt
European Patent Office
Office européen des brevets

(11) Publication number: **0 427 078 A1**

# (12) EUROPEAN PATENT APPLICATION

(21) Application number: **90120676.3**

(51) Int. Cl.[5]: **A61K 47/48**, A61K 31/505

(22) Date of filing: **29.10.90**

The title of the invention has been amended (Guidelines for Examination in the EPO, A-III, 7.3).

(30) Priority: **31.10.89 IT 2219989**

(43) Date of publication of application: **15.05.91 Bulletin 91/20**

(84) Designated Contracting States: **BE DE DK ES FR GB GR IT LU NL**

(71) Applicant: **MAGIS FARMACEUTICI S.p.A.**
**Via Cacciamali 34/36/38**
**I-25125 Brescia(IT)**

(72) Inventor: **Puricelli, Laura**
**Via Taramelli 7**
**I-25100 Brescia(IT)**

(74) Representative: **Gervasi, Gemma et al**
**NOTARBARTOLO & GERVASI Srl Viale Bianca Maria 33**
**I-20122 Milan(IT)**

## (54) Folinic acid-cyclodextrin inclusion compound.

(57) Complexes obtained by complexing folinic acid with α, β, γ, dimethyl-β or hydroxypropyl-β-cyclodextrin are described, having better activity in anemic syndromes due to folic acid deficiency than equivalent doses of the corresponding active principle.

Furthermore the stability of said compounds in an acid environment renders them suitable to be administered by oral route.



Xerox Copy Centre

# CYCLODEXTRIN COMPLEXES INDICATED FOR THE TREATMENT OF ALL ANEMIC FORMS DERIVING FROM FOLATE DEFICIENCY, THEIR PREPARATION AND RELATIVE PHARMACEUTICAL COMPOSITIONS

This invention relates to new compounds obtained by complexing folinic acid with a cyclodextrin chosen from α, β, γ, dimethyl-β and hydroxypropyl-β cyclodextrin, their preparation and the relative pharmaceutical compositions.

Folinic acid of formula (I)

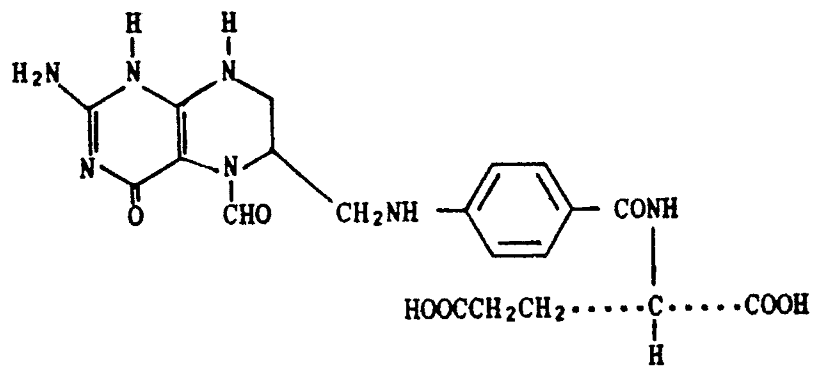

(I)

is used as an antidote for excessive doses of folic acid antagonists and to counteract the side effects induced by aminopterin (4-aminopteroylglutamic acid) and methotrexate (4-amino-$N^{10}$-methylpteroyl-glutamic acid).

Folinic acid is also indicated for all anemic forms deriving from folic acid deficiency due to increased requirement, reduced utilization, or insufficient dietary content of folates.

Folinic acid has however a drawback in that it is unstable in an acid environment, so that a reduction in its therapeutic activity can be encountered by oral administration of this active principle.

The present invention provides complexes of folinic acid of formula (I) or its pharmaceutically acceptable cation salts with a cyclodextrin chosen from α, β, γ, dimethyl-β and hydroxypropyl-β cyclodextrin.

In this respect it has been surprisingly found that the complexes according to the present invention are stable at acid pH, are rapidly absorbed and are better tolerated.

In the complexes according to the present invention the folinic acid and the cyclodextrin are present in a molar ratio of between 1:1 and 1:4.

Of the folinic acid salts used for preparing the complexes according to the present invention, calcium folinate is preferred.

The present invention also relates to the preparation of the complexes according to the present invention, which can be done by three different methods:

1) The folinic acid is dissolved directly in an aqueous solution of the chosen cyclodextrin and the solution obtained is stirred preferably at ambient temperature; the complex is separated by cold precipitation, preferably at a temperature of between 0 and 5 °C.

2) The compound and the chosen cyclodextrin are dissolved in a hot water/sodium hydroxide solution while stirring, and the complex is separated by lyophilization.

3) The calcium salt of folinic acid and the chosen cyclodextrin are dissolved in hot water while stirring, and the complex is separated by lyophilization or by spray drying.

The complexes of the present invention have more favourable properties than the drug used as such.

The new compounds have the same toxicity as calcium folinate ($LD_{50}$ greater than 7000 mg/kg in the mouse), but in compensation have superior antianemic activity and greater stability in an acid environment.

In particular, it has been found that if the new compounds are administered at a dose of 2 mg/kg to rats in whom anemia had already been induced by a folate-free milk diet, the activity in terms of restoration of normal erythrocyte concentration, influence on reticulocytic crises and influence on hemoglobin is higher than for calcium folinate administered as such at the same dose.

The following table shows the stability of the folinic acid-γ-cyclodextrin complex in a molar ratio of 1:3 compared with that of calcium folinate used as such, in solutions of pH 2.0.

TABLE

| STABILITY AT pH 2 | | | |
|---|---|---|---|
| MICROBIOLOGICAL TITRE | | | |
| DRUG | TIME 0 | AFTER 3 MONTHS | AFTER 6 MONTHS |
| Complex | 100% | 100% | 100% |
| Calcium folinate | 100% | 40% | 20% |

The present invention further provides pharmaceutical compositions for treating all forms of folate deficiency which are suitable for oral administration and contain as active principle the complexes of the present invention in combination with excipients normally used in pharmaceutical compositions.

Liquid oral compositions such as syrups (ready or extemporaneous) and solid oral compositions such as tablets, sugar coated pills, soft or hard capsules or single dose sachets are particularly suitable for the designated therapeutic use.

The pharmaceutical compositions according to the present invention can be administered once or twice per day in doses of between 5 and 15 mg depending on the gravity of the symptomatology.

The following examples are given as non-limiting illustration of the present invention.

EXAMPLE 1 The complex folinic acid-α-cyclodextrin in the molar ratio 1:1

4.734 g (0.01 moles) of folinic acid and 9.728 g (0.01 moles) of α-cyclodextrin are dissolved in 1 litre of water.

The solution is stirred for 3 hours at ambient temperature and then cooled to about +3°C. 14 g of product containing folic acid are obtained with a folinic acid concentration of about 32%.

EXAMPLE 2 The complex folinic acid-β-cyclodextrin in the molar ratio 1:2

22.64 g (0.02 moles) of β-cyclodextrin are suspended in 150 ml of water. 6.01 g (0.01 moles) of calcium folinate pentahydrate are then added at 60°C while stirring.

The mixture is stirred until a solution forms. The solution is dried by lyophilization or spray drying to obtain 26 g of a product with a folinic acid concentration of 17%.

EXAMPLE 3 The complex folinic acid-γ-cyclodextrin in the molar ratio 1:3

38.91 g (0.03 moles) of γ-cyclodextrin are suspended in 200 ml of water. 6.01 g (0.01 moles) of calcium folinate pentahydrate are then added at 60°C while stirring.

The mixture is stirred until a solution forms. The solution obtained is dried by lyophilization or spray drying.

About 45 g of product are obtained containing 10% by weight of folinic acid.

EXAMPLE 4 The complex folinic acid-dimethyl-β-cyclodextrin in the molar ratio 1:4

53.24 g (0.04 moles) of dimethyl-β-cyclodextrin are suspended in 300 ml of water. 6.01 g (0.01 moles) of calcium folinate pentahydrate are then added at 60°C while stirring.

The mixture is stirred until a solution forms. The solution obtained is dried by lyophilization or spray drying.

About 58 g of product are obtained containing 8% by weight of folinic acid.

EXAMPLE 5 The complex folinic acid-hydroxypropyl-β-cyclodextrin in the molar ratio 1:4

60 g (0.04 moles) of hydroxypropyl-β-cyclodextrin are suspended in 300 ml of water. 6.01 g (0.01 moles) of calcium folinate pentahydrate are then added at 60 °C while stirring.

The mixture is stirred until a solution forms. The solution obtained is dried by lyophilization or spray drying.

About 65 g of product are obtained containing 7.2% by weight of folinic acid.

## Claims

1. Complexes of folinic acid of formula (I)

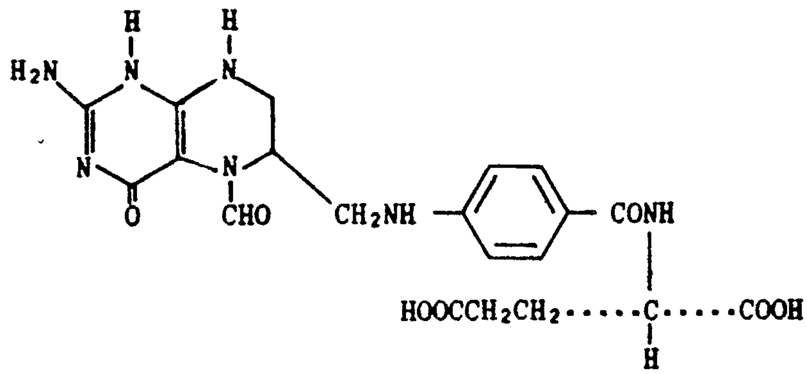

(I)

or its salts of pharmaceutically cations, with a cyclodextrin chosen from α, β, γ, dimethyl-β and hydroxypropyl-β-cyclodextrin.

2. The complexes claimed in claim 1, wherein folinic acid and the chosen cyclodextrin are present in a molar ratio of between 1:1 and 1:4.

3. The complexes claimed in claim 1, wherein the folinic acid is present in the form of its calcium salt.

4. A process for preparing the complexes claimed in claim 1, characterised by directly dissolving folinic acid in an aqueous solution of the chosen cyclodextrin and separating the complex by cold precipitation.

5. A process as claimed in claim 4, characterised in that the reaction is conducted at ambient temperature and the precipitation of the complex occurs at a temperature of between 0 and 5 °C.

6. A process for preparing the complexes claimed in claim 1, characterised by dissolving the compound and the chosen cyclodextrin in a hot water/sodium hydroxide solution while stirring, and separating the complex by lyophilization.

7. A process for preparing the complexes claimed in claim 1, characterised by dissolving the calcium salt of folinic acid and the chosen cyclodextrin in hot water while stirring, the complex being separated by lyophilization or by spray drying.

8. Pharmaceutical compositions suitable for oral administration for treating all anemic forms deriving from folate deficiency and containing as active principle the complexes of claim 1 in combination with excipients habitually used in pharmaceutical compositions.

9. Pharmaceutical compositions as claimed in claim 8 containing from 5 to 15 mg of active principle.

European Patent Office

# EUROPEAN SEARCH REPORT

Application Number

EP 90 12 0676

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | SCIENCE, vol. 232, no. 4754, 30th May 1986, pages 1132-1135; D.W. ARMSTRONG et al.: "Separation of drug stereisomers by the formation of beta-cyclodextrin inclusion complexes" * Whole article, in particular page 1134, column 2, line 2 * | 1-3 | A 61 K 47/48<br>A 61 K 31/505 |
| Y | IDEM | 1-9 | |
| Y | EP-A-0 295 476 (EDMOND PHARMA S.R.L.) * Whole document * | 4-9 | |
| Y | PATENT ABSTRACTS OF JAPAN, vol. 9, no. 28 (C-264)[1751], 6th February 1985; & JP-A-59 175 432 (YATORON K.K.) 04-10-1984 * Abstract * | 1-3 | |
| A | PATENT ABSTRACTS OF JAPAN, vol. 12, no. 172 (C-497)[3019], 21st May 1988; & JP-A-62 281 855 (DAIKIN IND. LTD) 07-12-1987 | | |

TECHNICAL FIELDS SEARCHED (Int. Cl.5)

A 61 K

The present search report has been drawn up for all claims

| Place of search | Date of completion of search | Examiner |
|---|---|---|
| The Hague | 06 February 91 | BENZ K.F. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document